# EUROPEAN PATENT APPLICATION

(11) **EP 4 840 459 A1**
(43) Date of publication of application: **18.11.2026**
(21) Application number: 24916954.1
(22) Date of filing: 26.12.2024
(51) Int. Cl.: A61K 38/16, A61P 19/02, C07K 2/00, C07K 14/52, C12N 15/12

(54) **AGENT FOR PREVENTING OR TREATING OSTEOARTHRITIS**

(30) Priority: 10.01.2024 JP 2024001674
(71) Applicant: The University of Osaka, Suita-shi, Osaka 565-0871 (JP)
(72) Inventor: EBINA, Kosuke, Suita-shi, Osaka 565-0871 (JP); SHIMAMURA, Munehisa, Suita-shi, Osaka 565-0871 (JP); NOGUCHI, Takaaki, Suita-shi, Osaka 565-0871 (JP); NAKATA, Ken, Suita-shi, Osaka 565-0871 (JP); OKADA, Seiji, Suita-shi, Osaka 565-0871 (JP); NAKAGAMI, Hironori, Suita-shi, Osaka 565-0871 (JP); MORISHITA, Ryuichi, Suita-shi, Osaka 565-0871 (JP)
(74) Representative: Meissner Bolte Partnerschaft mbB
(86) International application number: PCT/JP2024/046285
(87) International publication number: WO 2025/150453

(57) **Abstract**

An object is to provide a prophylactic or therapeutic agent for osteoarthritis capable of suppressing M1 polarization of macrophages. The object is achieved by a prophylactic or therapeutic agent for osteoarthritis containing an oligopeptide containing an amino acid sequence including a DE loop sequence of RANKL protein and a B-strand D sequence of the RANKL protein, the B-strand D sequence being located adjacent to the N-terminal side of the DE loop sequence.

## Description

### Technical Field

The present invention relates to a prophylactic or therapeutic agent for osteoarthritis and the like.

### Background Art

In a super-aging society, disorders of the locomotor system are the most frequent cause leading to a need for longterm care or support, and patients with knee osteoarthritis account for the largest number at approximately 25.3 million. Existing non-steroidal anti-inflammatory drugs (NSAIDs) and intra-articular hyaluronic acid administration provide certain analgesic effects, but are insufficient to suppress disease progression. Intra-articular steroid administration also raises concerns of cartilage necrosis.

NF-κB is a transcription factor that governs inflammation, immunity, cell differentiation, and some other related biological processes. In osteoarthritis, RANKL-RANK-NF-κB signaling promotes cartilage degeneration, and LPS-CD14 (expressed in macrophages etc., and functioning as a TLR)-NF-κB signaling induces arthritis mediated by macrophages etc., and promotes cartilage degeneration. Activation of Wnt signaling in cartilage also promotes cartilage degeneration. To date, steroids have been put into practical use as drugs that directly inhibit NF-κB. Steroids bind to intracellular glucocorticoid receptors (GR) and directly bind to NF-κB to inhibit the transcriptional activity, while GRs themselves act as a transcription factor, thereby causing various side effects (e.g., cartilage necrosis, osteoporosis, elevated blood glucose levels, and gastrointestinal ulcers).

PTL 1 reports that a RANKL peptide has an inhibitory effect on inflammatory cytokines via TLRs. However, there is no report on the prophylactic or therapeutic effect of RANKL peptides on osteoarthritis.

### Citation List

### Patent Literature

PTL 1: WO2016/186071A

### Non-patent Literature

NPL 1: The Journal of Clinical Investigation, No. 7, Vol. 108, 2001, pages 971-979.
NPL 2: Mol Endocrinol, January 2009, 23 (1): 35-46.

### Summary of Invention

### Technical Problem

One of the causes of osteoarthritis is M1 polarization of macrophages. It is presumed that M1 polarization serves as one of the contributing factors inducing exacerbation of synovitis, cartilage degeneration, osteosclerosis, osteoclast activation, and other related pathological conditions, thereby leading to the development and worsening of osteoarthritis.

An object of the present invention is to provide a prophylactic or therapeutic agent for osteoarthritis capable of suppressing M1 polarization of macrophages. Further, another object, whose achievement is more desirable although not essential, is to effectively prevent or treat osteoarthritis by suppressing multiple phenomena such as exacerbation of synovitis, cartilage degeneration, osteosclerosis, and osteoclast activation.

### Solution to Problem

As a result of diligent research in view of the objects above, the present inventors found that an oligopeptide composed of an amino acid sequence containing the DE loop sequence of RANKL protein and the β-strand D sequence of RANKL protein adjacent to the N-terminal side of the DE loop sequence can be used as an active ingredient of a prophylactic or therapeutic agent for osteoarthritis, capable of suppressing M1 polarization of macrophages. The inventors conducted further research based on this finding and completed the present invention. Specifically, the present invention includes the following aspects.

### Item 1.

A prophylactic or therapeutic agent for osteoarthritis, comprising
an oligopeptide comprising
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
   (a) the amino acid sequence of SEQ ID NO: 1, or
   (b) an amino acid sequence in which one amino acid is substituted, deleted, added, or inserted with respect to the amino acid sequence of SEQ ID NO: 1, and
the β-strand D sequence is the following (c) or (d):
   (c) the amino acid sequence of any of SEQ ID NOs: 2 to 5, or
   (d) an amino acid sequence in which one to three amino acids are substituted, deleted, added, or inserted with respect to the amino acid sequence of any of SEQ ID NOs: 2 to 5.

### Item 2.

The prophylactic or therapeutic agent according to Item 1, wherein the DE loop sequence is the amino acid sequence (a).

### Item 3.

The prophylactic or therapeutic agent according to Item 1 or 2, wherein the one to three amino acids are one or two amino acids.

### Item 4.

The prophylactic or therapeutic agent according to any one of Items 1 to 3, wherein in the amino acid sequence (d), the N-terminal leucine residue of SEQ ID NOs: 2 and 4 and the leucine residue that is the 4th amino acid residue from the N-terminus of SEQ ID NOs: 3 and 5 are not substituted or deleted.

### Item 5.

The prophylactic or therapeutic agent according to any one of Items 1 to 4, wherein the amino acid sequence of the oligopeptide contains a β-strand E sequence of the RANKL protein, the β-strand E sequence being located adjacent to the C-terminal side of the DE loop sequence.

### Item 6.

The prophylactic or therapeutic agent according to Item 5, wherein the β-strand E sequence is the following (e) or (f):
(e) the amino acid sequence of any of SEQ ID NOs: 6 to 9, or
(f) an amino acid sequence in which one to three amino acids are substituted, deleted, added, or inserted with respect to the amino acid sequence of any of SEQ ID NOs: 6 to 9.

### Item 7.

The prophylactic or therapeutic agent according to any one of Items 1 to 6, wherein the amino acid sequence of the oligopeptide does not comprise a CD loop sequence of the RANKL protein.

### Item 8.

The prophylactic or therapeutic agent according to any one of Items 1 to 7, wherein the amino acid sequence of the oligopeptide is the following (i) or (j):
(i) the amino acid sequence of any of SEQ ID NOs: 12 to 21, or
(j) an amino acid sequence in which one to three amino acids are substituted, deleted, added, or inserted with respect to the amino acid sequence of any of SEQ ID NOs: 12 to 21.

### Item 9.

The prophylactic or therapeutic agent according to any one of Items 1 to 7, wherein the amino acid sequence of the oligopeptide is the following (i') or (j'):
(i') the amino acid sequence of SEQ ID NO: 12, 17, 20, or 21, or
(j') an amino acid sequence in which one to three amino acids are substituted, deleted, added, or inserted with respect to the amino acid sequence of SEQ ID NO: 12, 17, 20, or 21.

### Item 10.

The prophylactic or therapeutic agent according to any one of Items 1 to 9, wherein the oligopeptide has a length of 50 amino acid residues or less.

### Item 11.

The prophylactic or therapeutic agent according to any one of Items 1 to 10, wherein the oligopeptide has a length of 40 amino acid residues or less.

### Item 12.

The prophylactic or therapeutic agent according to any one of Items 1 to 11, wherein the osteoarthritis is osteoarthritis in at least one joint selected from the group consisting of knee joint, shoulder joint, elbow joint, hip joint, ankle joint, and finger joint.

### Item 13.

An osteosclerosis inhibitor, comprising
an oligopeptide comprising
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
   (a) the amino acid sequence of SEQ ID NO: 1, or
   (b) an amino acid sequence in which one amino acid is substituted, deleted, added, or inserted with respect to the amino acid sequence of SEQ ID NO: 1, and
the β-strand D sequence is the following (c) or (d):
   (c) the amino acid sequence of any of SEQ ID NOs: 2 to 5, or
   (d) an amino acid sequence in which one to three amino acids are substituted, deleted, added, or inserted with respect to the amino acid sequence of any of SEQ ID NOs: 2 to 5.

### Item 14.

A method for preventing or treating osteoarthritis, comprising administering an oligopeptide to a patient with osteoarthritis,
wherein the oligopeptide comprises
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
   (a) the amino acid sequence of SEQ ID NO: 1, or
   (b) an amino acid sequence in which one amino acid is substituted, deleted, added, or inserted with respect to the amino acid sequence of SEQ ID NO: 1, and
the β-strand D sequence is the following (c) or (d):
   (c) the amino acid sequence of any of SEQ ID NOs: 2 to 5, or
   (d) an amino acid sequence in which one to three amino acids are substituted, deleted, added, or inserted with respect to the amino acid sequence of any of SEQ ID NOs: 2 to 5.

### Item 15.

A method for suppressing osteosclerosis, comprising administering an oligopeptide to a patient with osteoarthritis, wherein the oligopeptide comprises
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
   (a) the amino acid sequence of SEQ ID NO: 1, or
   (b) an amino acid sequence in which one amino acid is substituted, deleted, added, or inserted with respect to the amino acid sequence of SEQ ID NO: 1, and
the β-strand D sequence is the following (c) or (d):
   (c) the amino acid sequence of any of SEQ ID NOs: 2 to 5, or
   (d) an amino acid sequence in which one to three amino acids are substituted, deleted, added, or inserted with respect to the amino acid sequence of any of SEQ ID NOs: 2 to 5.

### Item 16.

An oligopeptide for use as a prophylactic or therapeutic agent for osteoarthritis, comprising
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
   (a) the amino acid sequence of SEQ ID NO: 1, or
   (b) an amino acid sequence in which one amino acid is substituted, deleted, added, or inserted with respect to the amino acid sequence of SEQ ID NO: 1, and
the β-strand D sequence is the following (c) or (d):
   (c) the amino acid sequence of any of SEQ ID NOs: 2 to 5, or
   (d) an amino acid sequence in which one to three amino acids are substituted, deleted, added, or inserted with respect to the amino acid sequence of any of SEQ ID NOs: 2 to 5.

### Item 17.

An oligopeptide for use as an osteosclerosis inhibitor, comprising an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
   (a) the amino acid sequence of SEQ ID NO: 1, or
   (b) an amino acid sequence in which one amino acid is substituted, deleted, added, or inserted with respect to the amino acid sequence of SEQ ID NO: 1, and
the β-strand D sequence is the following (c) or (d):
   (c) the amino acid sequence of any of SEQ ID NOs: 2 to 5, or
   (d) an amino acid sequence in which one to three amino acids are substituted, deleted, added, or inserted with respect to the amino acid sequence of any of SEQ ID NOs: 2 to 5.

### Item 18.

Use of an oligopeptide in the production of a prophylactic or therapeutic agent for osteoarthritis,
wherein the oligopeptide comprises
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
   (a) the amino acid sequence of SEQ ID NO: 1, or
   (b) an amino acid sequence in which one amino acid is substituted, deleted, added, or inserted with respect to the amino acid sequence of SEQ ID NO: 1, and
the β-strand D sequence is the following (c) or (d):
   (c) the amino acid sequence of any of SEQ ID NOs: 2 to 5, or
   (d) an amino acid sequence in which one to three amino acids are substituted, deleted, added, or inserted with respect to the amino acid sequence of any of SEQ ID NOs: 2 to 5.

### Item 19.

Use of an oligopeptide in the production of an osteosclerosis inhibitor,
wherein the oligopeptide comprises
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
   (a) the amino acid sequence of SEQ ID NO: 1, or
   (b) an amino acid sequence in which one amino acid is substituted, deleted, added, or inserted with respect to the amino acid sequence of SEQ ID NO: 1, and
the β-strand D sequence is the following (c) or (d):
   (c) the amino acid sequence of any of SEQ ID NOs: 2 to 5, or
   (d) an amino acid sequence in which one to three amino acids are substituted, deleted, added, or inserted with respect to the amino acid sequence of any of SEQ ID NOs: 2 to 5.

### Advantageous Effects of Invention

The present invention provides a prophylactic or therapeutic agent for osteoarthritis that suppresses M1 polarization of macrophages.

### Brief Description of Drawings

Fig. 1 shows the measurement results of OARSI score in Test Example 1. The vertical axis indicates OARSI score. In the horizontal axis, control indicates a sham surgery group, DMM indicates an untreated group of osteoarthritis models, and DMM+MHP1 indicates an MHP1-administered group of osteoarthritis models. The legend indicates the number of weeks elapsed from the sham surgery or the osteoarthritis model preparation surgery.
Fig. 2 shows the immunostaining results for type X collagen in Test Example 1. The vertical axis indicates the percentage of COL-X-positive cells in the articular surface. The horizontal axis is as explained in Fig. 1.
Fig. 3 shows the immunostaining results for IL-1β in Test Example 1. The vertical axis indicates the percentage of IL-1β-positive cells in the synovium. The horizontal axis is as explained in Fig. 1.
Fig. 4 shows the measurement results of synovitis score in Test Example 1. The vertical axis indicates synovitis score. The horizontal axis is as explained in Fig. 1.
Fig. 5 shows the immunostaining results of CD86 in Test Example 1. The vertical axis indicates the percentage of CD86-positive cells in the synovium. The horizontal axis is as explained in Fig. 1.
Fig. 6 shows the measurement results of BV/TV of subchondral bone in Test Example 1. The vertical axis indicates BV/TV (unit: %). The horizontal axis is as explained in Fig. 1.
Fig. 7 shows the immunostaining results for TRAP in Test Example 1. The vertical axis indicates the number of TRAP-positive cells (osteoclasts) per millimeter of a tibial subchondral bone section. The horizontal axis is as explained in Fig. 1.
Fig. 8 shows the immunostaining results for phosphorylated Smad2/3 in Test Example 1. The vertical axis indicates the number of phosphorylated Smad2/3-positive (TGF-β signal positive) cells per square millimeter of a tibial subchondral bone section. The horizontal axis is as explained in Fig. 1.
Fig. 9 shows the immunostaining results for CD31 in Test Example 1. The vertical axis indicates the number of CD31-positive cells per square millimeter of a tibial subchondral bone section (degree of angiogenesis). The horizontal axis is as explained in Fig. 1.
Fig. 10 shows the results of real-time PCR of LPS-stimulated mouse bone-marrow-derived macrophages in Test Example 2. The measured mRNA is shown above each graph. The vertical axis indicates the relative amount of mRNA. The horizontal axis indicates the administered drug and its dosage.
Fig. 11 shows the results of Western blotting of LPS-stimulated mouse bone-marrow-derived macrophages in Test Example 2. The measured proteins are shown on the left side of the photograph. The administered drug and its dosage are shown in the lower part of the photograph.
Fig. 12 shows the FACS analysis results of LPS-stimulated mouse bone-marrow-derived macrophages in Test Example 2. The administered drug is shown on the left side of each graph. The vertical axis of each graph indicates the expression level of a M2 marker, and the horizontal axis of each graph indicates the expression level of a M1 marker. The M1/M2 ratio is shown on the lower-left side of each graph.
Fig. 13 shows the TRAP staining results of mouse bone-marrow-derived macrophages that were induced to differentiate into osteoclasts in Test Example 2. In the graph, the vertical axis indicates the number of TRAP-positive cells, and the horizontal axis indicates the administered drug and its dosage.
Fig. 14 shows the results of real-time PCR of mouse bone-marrow-derived macrophages that were induced to differentiate into osteoclasts in Test Example 2. The measured mRNA is shown above each graph. The vertical axis indicates the relative amount of mRNA. The horizontal axis indicates the administered drug and its dosage.
Fig. 15 shows the results of Western blotting of mouse bone-marrow-derived macrophages that were induced to differentiate into osteoclasts in Test Example 2. The detected proteins are shown on the left side of the photograph. The administered drug and its dosage are shown on the upper side of the photograph.
Fig. 16 shows the results of Western blotting of TGF-β-stimulated bone-marrow-derived mesenchymal stem cells in Test Example 2. The detected proteins are shown on the left side of the photograph. The administered drug and its dosage are shown on the upper side of the photograph.
Fig. 17 shows the results of real-time PCR of TGF-β-stimulated bone-marrow-derived mesenchymal stem cells in Test Example 2. The measured mRNA is shown above each graph. The vertical axis indicates the relative amount of mRNA. The horizontal axis indicates the administered drug and its dosage.
Fig. 18 shows the results of ALP staining of bone-marrow-derived mesenchymal stem cells that were induced to differentiate into bone in the presence of TGF-β in Test Example 2. Fig. 18 also shows the results of real-time PCR at that time. The measured mRNA is shown above the graph. The vertical axis indicates the relative amount of mRNA. The horizontal axis indicates the administered drug and its dosage.
Fig. 19 shows the results of Alizarin red staining of bone-marrow-derived mesenchymal stem cells that were induced to differentiate into bone in the presence of TGF-β in Test Example 2. The absorbance at 450 nm at that time is also shown. The vertical axis indicates absorbance. The horizontal axis indicates the administered drug and its dosage.
Fig. 20 shows the results of a cell migration assay in human umbilical vein endothelial cells in Test Example 2. The quantitative evaluation is also shown. The vertical axis indicates cell migration. The horizontal axis indicates the administered drug and its dosage.
Fig. 21 shows the results of Western blotting of TGF-β-stimulated human umbilical vein endothelial cells in Test Example 2. The detected proteins are shown on the left side of the photograph. The administered drug and its dosage are shown at the top of the photograph. The quantitative results are also shown. The vertical axis indicates p-Smad2/Smad2. The horizontal axis indicates the administered drug and its dosage.

### Description of Embodiments

### 1. Definition

In the present specification, all amino acid sequences are represented by using the one-letter code.

In the present specification, the expressions "comprising" and "containing" include the concepts of comprising, containing, consisting essentially of, and consisting of.

The "identity" of amino acid sequences refers to the degree of match between two or more comparable amino acid sequences. Thus, the higher the degree of match between two amino acid sequences, the higher the identity or similarity of those sequences. The level of amino acid sequence identity can be determined, for example, by using a sequence analysis tool, such as FASTA, with default parameters. Alternatively, the level of amino acid sequence identity can be determined using the BLAST algorithm by Karlin and Altschul (Karlin S., Altschul S. F. Methods for assessing the statistical significance of molecular sequence features by using general scoring schemes, Proc Natl Acad Sci USA. 87: 2264-2268 (1990); and Karlin S., Altschul S. F., Applications and statistics for multiple high-scoring segments in molecular sequences, Proc Natl Acad Sci USA. 90: 5873-7 (1993)). A program called "BLASTX," based on such a BLAST algorithm, has been developed. The specific techniques of these analysis methods are known and can be found on the website of the National Center of Biotechnology Information (NCBI) (http://www.ncbi.nlm.nih.gov/). The identity of base sequences is also defined in the same manner as above.

In the present specification, "conservative substitution" means that an amino acid residue is substituted with another amino acid residue having a similar side chain. For example, the substitution between amino acid residues having a basic side chain, such as lysine, arginine, or histidine, is considered to be a conservative substitution. The following substitutions between other amino acid residues are also considered to be a conservative substitution: the substitution between amino acid residues having an acidic side chain such as aspartic acid or glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

### 2. Oligopeptide

The following describes an oligopeptide composed of an amino acid sequence containing the DE loop sequence of RANKL protein and the B-strand D sequence of the RANKL protein located adjacent to the N-terminal side of the DE loop sequence (which is also referred to as "the oligopeptide of the present invention" in the present specification).

The DE loop sequence of the RANKL protein may be the following amino acid sequence (a) or (b):
(a) the amino acid sequence of SEQ ID NO: 1, or
(b) an amino acid sequence in which one amino acid is substituted, deleted, added, or inserted with respect to the amino acid sequence of SEQ ID NO: 1.

Preferably, the substitution in the amino acid sequence (b) is a conservative substitution. Preferably, the addition in the amino acid sequence (b) is an addition at the N-terminus or C-terminus.

The β-strand D sequence of RANKL protein is not particularly limited as long as it is an amino acid sequence forming β-strand D within the amino acid sequence of RANKL protein. The species from which the β-strand D sequence is originated is not particularly limited, and examples include various mammals, such as humans, monkeys, mice, rats, dogs, cats, and rabbits. Of these, the species is preferably humans, monkeys, mice, rats, and the like, more preferably humans, mice and the like, and even more preferably humans. The β-strand D sequence of individual species is publicly known; for example, in the case of mice and rats, their β-strand D sequences are disclosed in NPL 1, NPL 2, or other documents. Even if the β-strand D sequence originated from a certain species is not publicly known, the sequence can be easily determined based on publicly known information (e.g., NPL 1 and NPL 2). Specific examples of the β-strand D sequence of RANKL protein include the amino acid sequence of SEQ ID NO: 2 (a portion of the β-strand D sequence of mouse-derived RANKL protein), the amino acid sequence of SEQ ID NO: 3 (the β-strand D sequence of mouse-derived RANKL protein), the amino acid sequence of SEQ ID NO: 4 (a portion of the β-strand D sequence of human-derived RANKL protein), and the amino acid sequence of SEQ ID NO: 5 (the β-strand D sequence of human-derived RANKL protein). The β-strand D sequence may be mutated as long as the oligopeptide of the present invention can exert effects, such as suppression of M1 polarization of macrophages.

In SEQ ID NOs: 2 to 5, the leucine residue at the N-terminus of SEQ ID NOs: 2 and 4 and the leucine residue that is the fourth amino acid from the N-terminus of SEQ ID NOs: 3 and 5 are important for exerting effects such as suppression of M1 polarization of macrophages. In the β-strand D sequence, it is desirable that these leucine residues are not mutated.

The β-strand D sequence of RANKL protein may be preferably the following amino acid sequence (c) or (d):
(c) the amino acid sequence of any one of SEQ ID NOs: 2 to 5, or
(d) an amino acid sequence in which one to three amino acids are substituted, deleted, added, or inserted with respect to the amino acid sequence of any of SEQ ID NOs: 2 to 5.

In the amino acid sequence (d), the number of mutated (substituted, deleted, added, or inserted) amino acids is preferably one or two, more preferably one.

For the amino acid sequence (d), conservative substitution can be mentioned as a preferable mutation. Further, the amino acid sequence (d) preferably includes the following amino acid sequence (d'):
(d') an amino acid sequence in which one to three amino acids are added to the N-terminus or C-terminus (preferably the C-terminus) of the amino acid sequence of any of SEQ ID NOs: 2 to 5.

In the amino acid sequence (d'), the number of amino acids added is preferably one or two, more preferably one.

The phrase "located adjacent to the N-terminal side" means that the amino acid at the N-terminus of the DE loop sequence and the amino acid at the C-terminus of the β-strand D sequence are linked via a peptide bond.

The amino acid sequence of the oligopeptide of the present invention may include sequences other than those described above, as long as the oligopeptide of the present invention can exhibit effects such as suppression of M1 polarization of macrophages. The other sequences are not particularly limited, but are desirably determined from the viewpoint of, for example, a longer intracellular half-life. Whether a sequence is hydrophilic and its intracellular half-life can be predicted on various websites (e.g., ExPASy (http://web.expasy.org/protparam/)). When using ExPASy, it is preferable to design the sequence so that the "Grand average of hydropathicity" shows a negative value in regards to hydrophilicity.

As another sequence, the β-strand E sequence of RANKL protein is particularly preferable from the viewpoint of the suppression effect on M1 polarization of macrophages and the like. The β-strand E sequence is preferably located adjacent to the C-terminal side of the DE loop sequence. The phrase "adjacent to the C-terminal side" indicates that the amino acid at the C-terminus of the DE loop sequence and the amino acid at the N-terminus of the β-strand E sequence are linked via a peptide bond.

The β-strand E sequence of RANKL protein is not particularly limited as long as it is an amino acid sequence forming β-strand E within the amino acid sequence of RANKL protein. The species from which the β-strand E sequence is originated is not particularly limited, and examples include various mammals, such as humans, monkeys, mice, rats, dogs, cats, and rabbits. Of these, the species is preferably humans, monkeys, mice, rats, and the like, more preferably humans, mice and the like, and even more preferably humans. The β-strand E sequence of individual species is publicly known; for example, in the case of mice and rats, their β-strand E sequences are disclosed in NPL 1, NPL 2, or other documents. Even if the β-strand E sequence originated from a certain species is not publicly known, the sequence can be easily determined based on publicly known information (e.g., NPL 1 and NPL 2). Specific examples of the β-strand E sequence of RANKL protein include the amino acid sequence of SEQ ID NO: 6 (the β-strand E sequence of mouse-derived RANKL protein), the amino acid sequence of SEQ ID NO: 7 (a portion of the β-strand E sequence of mouse-derived RANKL protein), the amino acid sequence of SEQ ID NO: 8 (the β-strand E sequence of human-derived RANKL protein), and the amino acid sequence of SEQ ID NO: 9 (a portion of the β-strand E sequence of human-derived RANKL protein). The β-strand E sequence may be mutated as long as the oligopeptide of the present invention can exert effects, such as suppression of M1 polarization of macrophages.

The β-strand E sequence of RANKL protein may be preferably the following amino acid sequence (e) or (f):
(e) the amino acid sequence of any of SEQ ID NOs: 6 to 9, or
(f) an amino acid sequence in which one to three amino acids are substituted, deleted, added, or inserted with respect to the amino acid sequence of any of SEQ ID NOs: 6 to 9.

In the amino acid sequence (f), the number of mutated (substituted, deleted, added, or inserted) amino acids is preferably one or two, more preferably one.

In the amino acid sequence (f), conservative substitution can be mentioned as a preferable mutation. Further, the amino acid sequence (f) preferably includes the following amino acid sequence (f'):
(f') an amino acid sequence in which one to three amino acids are added to the N-terminus or C-terminus (preferably N-terminus) of the amino acid sequence of any of SEQ ID NOs: 6 to 9.

In the amino acid sequence (f'), the number of amino acids added is preferably one or two, more preferably one.

The amino acid sequence of the oligopeptide of the present invention preferably does not include the CD loop sequence of RANKL protein.

The CD loop sequence of RANKL protein is an amino acid sequence that forms a CD loop within the amino acid sequence of RANKL protein. The CD loop sequence of individual species is publicly known; for example, in the case of mice and rats, their CD loop sequences are disclosed in NPL 1, NPL 2, or other documents. Specific examples of the CD loop sequence of RANKL protein include the amino acid sequence of SEQ ID NO: 10 (the CD loop sequence of mouse-derived RANKL protein), and the amino acid sequence of SEQ ID NO: 11 (the CD loop sequence of human-derived RANKL protein).

The length of the oligopeptide of the present invention is not particularly limited as long as it is a typical length as an oligopeptide. The length is, for example, 50 amino acid residues or less, preferably 40 amino acid residues or less, more preferably 35 amino acid residues or less, more preferably 30 amino acid residues or less, and even more preferably 25 amino acid residues or less. The length is also, for example, 11 amino acid residues or more, preferably 13 amino acid residues or more, more preferably 15 amino acid residues or more, even more preferably 20 amino acid residues or more, yet even more preferably 25 amino acid residues or more. The range of length is, for example, 11 to 50 amino acid residues, preferably 15 to 40 amino acid residues, more preferably 20 to 35 amino acid residues, even more preferably 25 to 30 amino acid residues.

Preferred amino acid sequences of the oligopeptide of the present invention include an oligopeptide containing the following amino acid sequence (i) or (j):
(i) the amino acid sequence of any of SEQ ID NOs: 12 to 21, or
(j) an amino acid sequence in which one to three amino acids are substituted, deleted, added, or inserted with respect to the amino acid sequence of any of SEQ ID NOs: 12 to 21.

More preferable amino acid sequences of the oligopeptide of the present invention include oligopeptides containing the following amino acid sequence (i') or (j'):
(i') the amino acid sequence of SEQ ID NO: 12, 17, 20, or 21, or
(j') an amino acid sequence in which one to three amino acids are substituted, deleted, added, or inserted with respect to the amino acid sequence of SEQ ID NO: 12, 17, 20, or 21.

In the amino acid sequences (j) and (j'), the number of mutated (substituted, deleted, added, or inserted) amino acids is preferably one or two, more preferably one.

Preferably, the substitution in the amino acid sequences (j) and (j') is a conservative substitution.

The oligopeptide of the present invention may be chemically modified as long as it can exert effects such as suppression of M1 polarization of macrophages.

In the oligopeptide of the present invention, the C-terminus may be any of a carboxyl group (-COOH), a carboxylate (-COO⁻), an amide (-CONH₂), or an ester (-COOR).

For R in the ester, the following can be used: for example, a C₁₋₆ alkyl group, such as methyl, ethyl, n-propyl, isopropyl, and n-butyl; for example, a C₃₋₈ cycloalkyl group, such as cyclopentyl and cyclohexyl; for example, a C₆₋₁₂ aryl group, such as phenyl and α-naphthyl; for example, a phenyl-C₁₋₂ alkyl group, such as benzyl and phenethyl; a C₇₋₁₄ aralkyl group, such as an α-naphthyl-C₁₋₂ alkyl group, such as α-naphthylmethyl; and a pivaloyloxymethyl group.

Further, the oligopeptide of the present invention also encompasses oligopeptides in which the amino group of the amino acid residue at the N-terminus is protected with a protecting group (e.g., a C₁₋₆ acyl group, such as a C₁₋₆ alkanoyl group, including a formyl group and an acetyl group).

Among the chemical modifications above, amidation of the C-terminus and protection of the N-terminus with an acetyl group are preferred. In particular, oligopeptides having an acetylated N-terminus have excellent activity and are thus preferred. From the viewpoint of stability, an oligopeptide having an amidated C-terminus is excellent. More preferred oligopeptides include an oligopeptide having an amidated C-terminus and an acetylated N-terminus. Particularly preferred oligopeptides include MHP1-7, which is the oligopeptide of the present invention represented by SEQ ID NO: 12 (MHP1) with the C-terminus amidated and the N-terminus acetylated, MHP6-AcN, which is MHP6 with the C-terminus amidated and the N-terminus acetylated, MHP24-AcN, which is MHP24 with the C-terminus amidated and the N-terminus acetylated, and MHP24h-AcN, which is MHP24h with the C-terminus amidated and the N-terminus acetylated.

Furthermore, the oligopeptide of the present invention may have a known modifier added for the purpose of improving drug stability, pharmacokinetics, bioavailability, and the like. Examples of such modifiers include a polyethylene glycol chain.

The oligopeptide of the present invention encompasses those of various forms, such as linear, branched, or cyclic oligopeptides, but is preferably a linear oligopeptide. Further, the oligopeptide of the present invention may be crosslinked according to or based on a known means, as long as the oligopeptide can exert M1 polarization of macrophages and the like.

The oligopeptide of the present invention may be in the form of a pharmaceutically acceptable salt formed with an acid or a base. The salt is not particularly limited as long as it is a pharmaceutically acceptable salt, and either an acidic salt or a basic salt can be used. Examples of acid salts include inorganic acid salts, such as hydrochloride, hydrobromide, sulfate, nitrate, and phosphate; organic acid salts, such as acetate, propionate, tartrate, fumarate, maleate, malate, citrate, methanesulfonate, and p-toluenesulfonate; and amino acid salts, such as aspartate and glutamate. Examples of basic salts include alkali metal salts, such as sodium salts and potassium salts; and alkaline earth metal salts, such as calcium salts and magnesium salts.

The oligopeptide of the present invention may be in the form of a solvate. The solvent is not particularly limited as long as it is pharmaceutically acceptable. Examples include water, ethanol, glycerol, and acetic acid.

The oligopeptide of the present invention can be produced according to a known peptide synthesis method depending on its amino acid sequence.

### 3. Use

The oligopeptide of the present invention can suppress M1 polarization of macrophages and can be used as an active ingredient of a prophylactic or therapeutic agent for osteoarthritis. In addition to the effect of suppressing M1 polarization of macrophages, the oligopeptide of the present invention can also be used as an active ingredient in drugs aimed at the effects such as the effect of suppressing cartilage degeneration, the effect of suppressing changes in subchondral bone, the effect of suppressing inflammation at the articular surface, the effect of suppressing synovitis, the effect of suppressing infiltration of macrophages, the effect of suppressing osteosclerosis of subchondral bone, the effect of suppressing osteoclast differentiation, the effect of suppressing TGF-β signaling, the effect of suppressing angiogenesis in subchondral bone, the effect of suppressing migration of vascular endothelial cells, and the like, in osteoarthritis. Furthermore, the oligopeptide of the present invention can also be used as an active ingredient of an osteosclerosis inhibitor.

In the present specification, the agents described above may be collectively referred to as "the agent of the present invention." The agent of the present invention can be an agent used in various fields such as pharmaceuticals. The agent of the present invention may be applied to animals and humans either as such or in the form of various compositions containing commonly used components (e.g., by administration, ingestion, or injection).

For the agent of the present invention, the oligopeptide of the present invention may be used singly or in a combination of two or more kinds.

The osteoarthritis is, for example, osteoarthritis in at least one joint selected from the group consisting of knee joint, shoulder joint, elbow joint, hip joint, ankle joint, and finger joint. The osteoarthritis can also be, for example, primary osteoarthritis or secondary osteoarthritis.

In the present specification, "treatment" can include concepts such as cure, remission, alleviation, relief, and suppression of progression of symptoms. Additionally, "prevention" can include not only preventing onset, but also concepts such as delaying onset and suppressing symptoms when a disease has developed.

The content of the active ingredient in the agent of the present invention can be appropriately set in consideration of the type of target disease, the intended therapeutic effect, the administration method, the treatment period, the age of the patient, the body weight of the patient, and the like. For example, the content of the active ingredient in the agent of the present invention can be about 0.0001 parts by weight to 100 parts by weight, based on the entire agent of the present invention taken as 100 parts by weight.

The manner of administration of the agent of the present invention is not particularly limited as long as the desired effect is obtained, and the agent can be administered to mammals, including humans, via any administration route, either oral administration or parenteral administration (e.g., intravenous injection, intramuscular injection, subcutaneous administration, rectal administration, transdermal administration, and topical administration). The manner of administration is preferably parenteral administration. Dosage forms for oral administration and parenteral administration and methods for producing those forms are well known to those skilled in the art. Such dosage forms can be produced according to commonly used methods, for example, by mixing the active ingredient with a pharmaceutically acceptable carrier and the like.

The dosage forms for parenteral administration include injectable formulations (e.g., intravenous infusions, intravenous injections, intramuscular injections, subcutaneous injections, and intradermal injections), formulations for external use (e.g., ointments, cataplasms, lotions, creams, and gels), suppositories, inhalants, eye drops, ophthalmic ointments, nasal drops, ear drops, and liposome agents. For example, an injectable formulation is prepared by dissolving the oligopeptide of the present invention in distilled water for injection; optionally, a solubilizer, a buffer, a pH adjuster, an isotonic agent, a soothing agent, a preservative, a stabilizer, etc. may be added. The agent of the present invention can also be in the form of a lyophilized formulation for on-demand preparation.

The agent of the present invention may further contain other drugs effective for treatment or prevention of diseases. The agent of the present invention may also contain components such as bactericides, anti-inflammatory agents, cell activators, vitamins, and amino acids as needed.

The carrier for use in formulating the agent of the present invention may contain excipients, binders, disintegrants, lubricants, colorants, and taste-masking and/or odor-masking agents commonly used in the art, and also may optionally contain stabilizers, emulsifiers, absorption enhancers, surfactants, pH adjusters, antiseptics, antioxidants, bulking agents, wetting agents, surface activators, dispersants, buffers, preservatives, solubilizers, soothing agents, and the like.

The dosage of the agent of the present invention can be determined, for example, based on various factors, such as the route of administration, the type of disease, the degree of symptoms, the age, sex, and body weight of the patient, the severity of disease, pharmacological findings such as pharmacokinetic and toxicological characteristics, the use or nonuse of a drug delivery system, and whether the agent is administered as part of a combination with other drugs. The dosage of the agent of the present invention can be, for example, about 1 µg/kg (body weight) to 10 g/kg (body weight) per day. The administration schedule for the agent of the present invention can also be determined by taking into account the same factors as those for the dosage. For example, the agent of the present invention may be administered at the above-described daily dose one to five times over a period of from one day to one month.

### Examples

The present invention is described in detail below based on Examples. However, the present invention is not limited to these Examples.

### Production Example 1: Synthesis of Oligopeptide

Oligopeptides having the amino acid sequences shown in Table 1 were synthesized by ILS Inc. on an outsourced basis. Except for MHP24-AcN and MHP24h-AcN, the N-terminus and C-terminus were unmodified; i.e., the N-terminus was an amino group, and the C-terminus was a carboxyl group. MHP24-AcN and MHP24h-AcN are oligopeptides having the N-terminus acetylated and the C-terminus amidated. In these peptides, all amino acid residues are in the L-form.

Furthermore, synthesis of the following oligopeptides was outsourced to ILS Inc.: an oligopeptide formed by substitution of the methionine residue of MHP1 with D-methionine (MHP1-3), an oligopeptide formed by N-terminal acetylation of MHP1 (MHP1-6), an oligopeptide formed by N-terminal acetylation of MHP1 and C-terminal amidation (MHP1-7 or MHP1-AcN), and an oligopeptide formed by C-terminal amidation of MHP1 (MHP1-8).

Furthermore, synthesis of an oligopeptide obtained by N-terminal acetylation of MHP1 and by addition of a biocytin (biotinylated lysine) residue to the C-terminus (biotinylated MHP1) was outsourced to Bachem Inc.

HPLC and MS indicated that oligopeptides having desired sequences were synthesized with high purity. These peptides may be collectively referred to below as "RANKL peptide."

**Table 1**

| **Name** | **Sequence** | **Species of Origin** | **Number of Amino Acids** | **SEQ ID NO** |
|---|---|---|---|---|
| **MHP1** | **LMVYWKTSIKIPSSHNLMKGGSTKNWSGN** | **Mouse** | **30** | **12** |
| **MHP2** | **DYLQLMVYVVKTSIKIPSSHNLMKGGSTKN** | **Mouse** | **30** | **13** |
| **MHP4** | **LMVYWKTSIKIPSS** | **Mouse** | **15** | **14** |
| **hMHP4** | **LMVYVTKTSIKIPSS** | **Human** | **15** | **15** |
| **MHP5** | **LMVYVVKTSIKIPSSHNLMKGG** | **Mouse** | **22** | **16** |
| **MHP6** | **LMVYVTKTSIKIPSSHTLMKGGSTKYWSGN** | **Human** | **30** | **17** |
| **MHP12** | **LMVYVVKTSIKIPSSHNLMKGGS** | **Mouse** | **23** | **18** |
| **MHP13** | **LMVYVVKTSIKIPSSHNLMKGGSTKN** | **Mouse** | **26** | **19** |
| **MHP24 -AcN** | **LMVYWKTSIKIPSSHNLMKGGST** | **Mouse** | **24** | **20** |
| **MHP24h -AcN** | **LMVYVTKTSIKIPSSHTLMKGGST** | **Human** | **24** | **21** |

### Test Example 1: In Vivo Test

### 1-1: Preparation of Animal Models and Administration of RANKL Peptide

Osteoarthritis models with destabilization of the medial meniscus (DMM) were prepared using C57BJ/6 mice (male, 8 to 10 weeks old). Specifically, the following procedure was performed.

After analgesia and sedation were achieved by intraperitoneal administration of triple mixed anesthesia (medetomidine, vetorphale, and midazolam), a 1.5 cm longitudinal incision was made on the medial side of the knee joint with a scalpel. Additionally, the vastus medialis was longitudinally incised proximally from the medial side of the patellar tendon, and the patella was dislocated laterally to gain entry into the joint. The meniscotibial ligament connecting the medial meniscus to the tibia was severed on the anterior side of the tibia with a scalpel. Finally, the patella was reduced, and the joint capsule and skin were sutured with 5-0 nylon thread. In this surgery, destabilization of the medial meniscus allows onset of osteoarthritis of the knee joint through a more physiological mechanism. Degeneration of cartilage was observed from 2 weeks after surgery, and knee osteoarthritis was completed in about 8 weeks. Joint pain and cartilage degeneration observed in knee osteoarthritis were reproduced. From the day of surgery, MHP1 dissolved in 0.45% NaCl was administered intraperitoneally at 200 µl (600 µg) five times per week, and comparison was made with a 0.45% NaCl-administered group (untreated group). To a sham surgery group as well, 0.45% NaCl was administered. The following evaluations were performed at 2 weeks, 4 weeks, and 8 weeks after surgery.

### 1-2: Histological Evaluation

Mice were euthanized, and the knee joints were excised by transecting the femur and tibia at their midpoints. After fixation with formalin, coronal sections (5 µm) of paraffin blocks were prepared, followed by performing hematoxylin-eosin staining, safranin O staining, and immunostaining (type X collagen, IL-1β, CD86, TRAP, phosphorylated Smad2/3) to evaluate tissues such as cartilage matrix, subchondral bone, and osteophyte formation. The synovial tissue was evaluated using the synovitis score proposed by Krenn et al., and the cartilage tissue was evaluated using the O'Driscoll score and the OARSI score.

### 1-3: Results

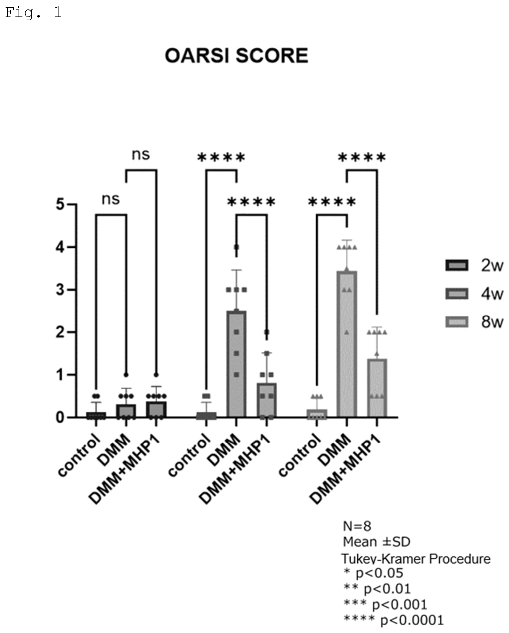

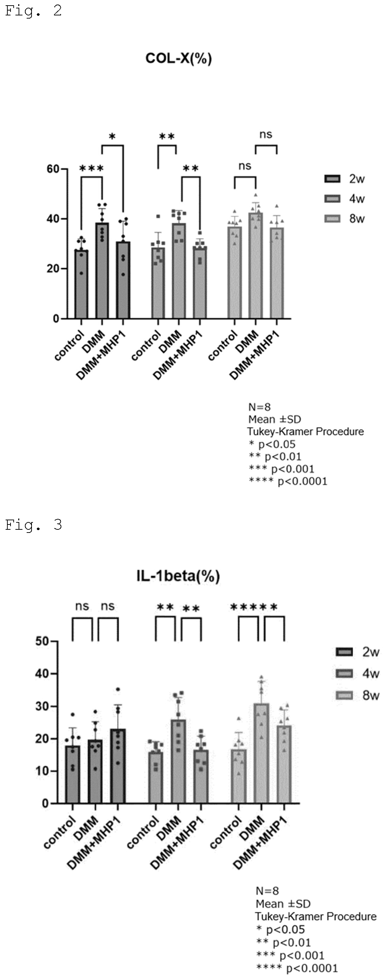

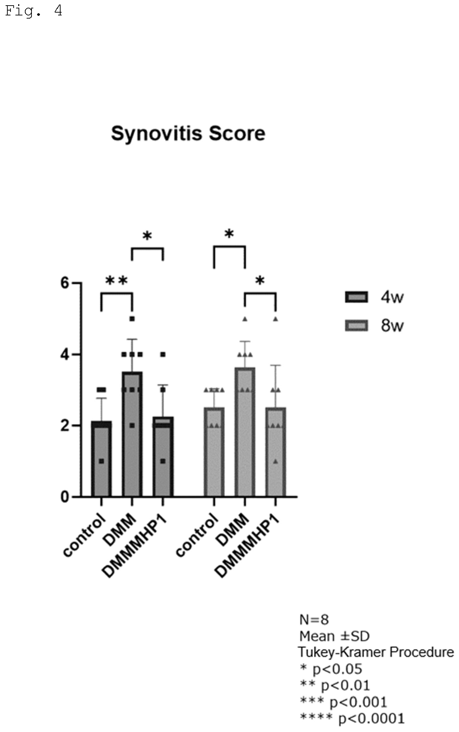

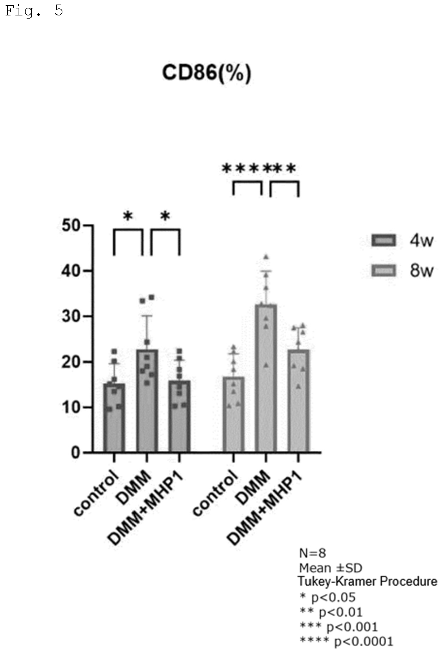

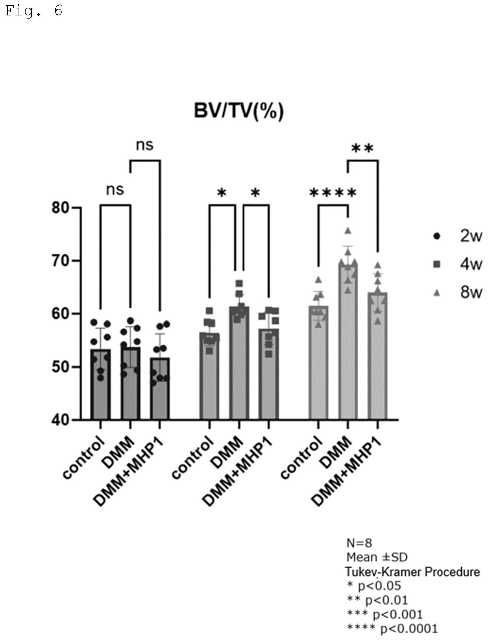

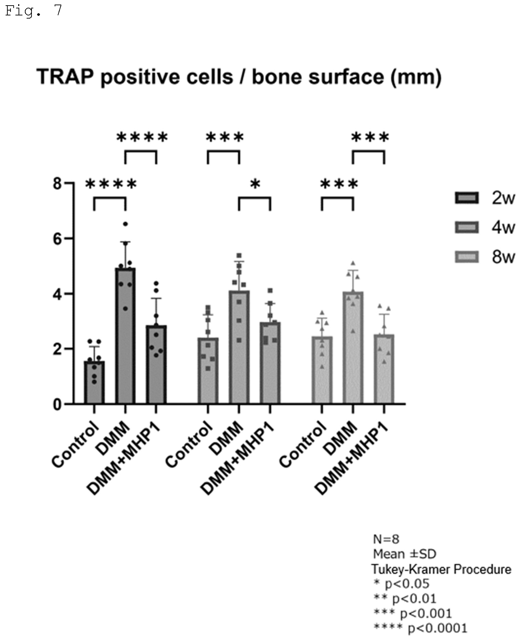

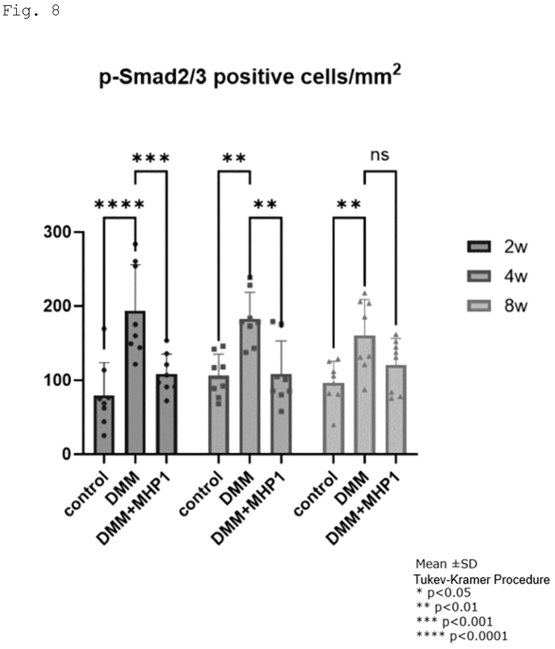

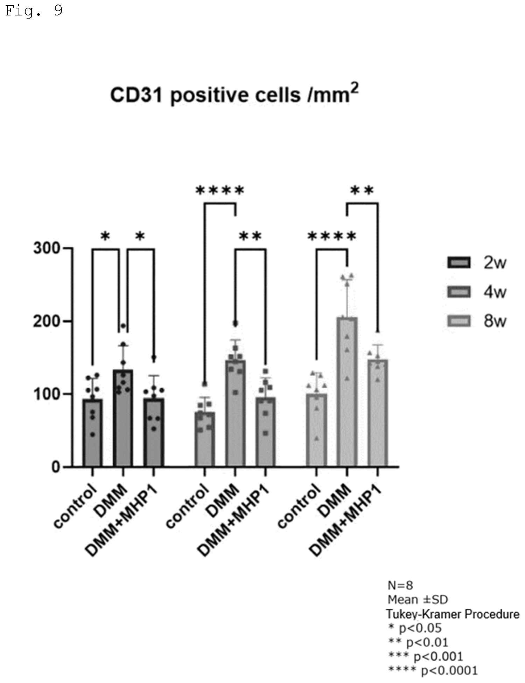

Figs. 1 to 9 show the results. Fig. 1 indicated that the RANKL peptide suppresses cartilage degeneration in osteoarthritis. Fig. 2 indicated that the RANKL peptide suppresses changes in subchondral bone in osteoarthritis. Fig. 3 indicated that the RANKL peptide suppresses inflammation in the articular surface in osteoarthritis. Fig. 4 indicated that the RANKL peptide suppresses synovitis in osteoarthritis. Fig. 5 indicated that the RANKL peptide suppresses macrophage infiltration in osteoarthritis. Fig. 6 indicated that the RANKL peptide suppresses osteosclerosis (increased bone density) of subchondral bone in osteoarthritis. Fig. 7 indicated that the RANKL peptide reduces the number of osteoclasts in the tibial subchondral bone in osteoarthritis. Fig. 8 indicated that the RANKL peptide suppresses TGF-β signaling in the tibial subchondral bone in osteoarthritis. Fig. 9 indicated that the RANKL peptide suppresses angiogenesis in the tibial subchondral bone in osteoarthritis.

### Test Example 2: In Vitro Test

### 2-1: Evaluation of Apoptosis, Inflammatory Cytokine Production, and Matrix-degrading Enzyme Production of Chondrocytes and Synoviocytes

Mouse primary chondrocytes were collected from the knee joint surface of 5-day-old C57B/6J mice, and used in experiments. For human synoviocytes, the synovium was collected during artificial knee replacement, and synovium-derived mesenchymal stem cells were used in experiments.

The cells were seeded in a 96-well plate at a concentration of 4 × 10³ cells/well, and cultured for 24 hours. Thereafter, the MHP1 peptide was added at a concentration of 4 mg/ml (25 µl per well), and the cells were cultured for an additional 2 hours. From this point in time, IL-1β (10 ng/ml) was added at 100 µl/well, and a viable cell count reagent SF was administered at 24 hours, 48 hours, and 72 hours after administration. After 2 hours from each time point, absorbance was measured to evaluate apoptosis.

Further, the cells above (8 × 10⁴) were seeded in a 24-well plate and cultured for 24 hours. Thereafter, the MHP1 peptide was added at a concentration of 4 mg/ml (25 µl per well), and the cells were cultured for an additional 2 hours. From this point in time, IL-1β (10 ng/ml) was added at 100 µl/well, and the cells were collected after 24 hours. Quantitative evaluation of inflammatory cytokines such as IL-6 and TNF-α and extracellular matrix-degrading enzymes such as MMP-3, MMP-13, ADAMTS-4, and ADAMTS-5 was performed by real-time PCR and Western blotting.

### 2-2: Evaluation of Macrophage Polarization and Evaluation of Anti-inflammatory Action

Macrophages are known to polarize into subtype M1, which induces inflammatory responses according to surrounding conditions, and subtype M2, and are considered to play an important role in the homeostasis of articular cartilage.

Mouse bone-marrow-derived macrophages ("BMDMs" below) were prepared. Bone marrow contents were collected from the femoral and tibial diaphyses of C57BL/6J mice (8 weeks old), and blood cell components were removed therefrom, followed by stimulating the remainder with M-CSF to obtain BMDMs.

BMDMs were seeded in a 24-well plate at a concentration of 8.0 × 10⁴ cell/well and cultured for 24 hours. Thereafter, the MHP1 peptide was added at a concentration of 4 mg/ml (25 µl per well), and the cells were cultured for 2 hours. Further, the cells were stimulated with inflammatory cytokines such as LPS and IL-1β, and collected after 24 hours. The expressions of CD86 and iNOS, characteristic of subtype M1, and CD206, IL-10, and the like, characteristic of subtype M2, were evaluated by real-time PCR and Western blotting. Additionally, anti-inflammatory action was also evaluated based on the changes in expression of inflammatory cytokines such as IL-6 and TNF-α. Furthermore, FACS analysis was performed using M1 and M2 markers.

### 2-3: Evaluation of the Suppression of Macrophage Differentiation into Osteoclasts

BMDMs were prepared according to the same method as above.

2-3-1
BMDMs were seeded in a 96-well plate at a concentration of 2.0 × 10⁵/well. M-CSF (10 ng/ml), RANKL, and MHP1 were added at respective concentrations to induce differentiation into osteoclasts. Formation of osteoclasts was confirmed on day 4 after seeding in the 96-well plate, and then TRAP staining was performed.

2-3-2
BMDMs were seeded in a 24-well plate at a concentration of 8.0 × 10⁴/well. M-CSF (10 ng/ml), RANKL (50 ng/ml), and MHP1 were added at respective concentrations to induce differentiation into osteoclasts. The cells were collected on day 4 after the start of administration, and the mRNA expression levels of TRAP, MMP9, DCSTAMP, Cathepsin-K, and TRAF6 were evaluated by real-time PCR.

2-3-3
BMDMs were seeded in a 6-well plate at a concentration of 1.0 × 10⁶/well. M-CSF (10 ng/ml), RANKL (50 ng/ml), and MHP1 were added at respective concentrations to induce differentiation into osteoclasts. The cells were collected on day 4 and evaluated for p-p65, p65, p-ERK, ERK, p-p38, p38, p-IKKα, and p52 by Western blotting.

2-3-4
Bone marrow contents were collected from the femoral and tibial diaphyses of C57BL/6J mice (8 weeks old), and blood cell components and macrophage components were removed therefrom to obtain bone-marrow-derived mesenchymal stem cells. The cells were seeded in a 24-well plate at a concentration of 8.0 × 10⁴ cells/well, and the MHP1 peptide was added at a concentration of 4 mg/ml (25 µl per well), followed by culturing the cells for 24 hours. TGF-β was further added at a concentration of 2 ng/ml, and the cells were collected after 1 hour. Evaluation was performed on p-smad2 and smad2 by Western blotting.

MHP1 was also added at different concentrations, and the cells were collected after 24 hours, followed by evaluating TGFβ receptors 1 and 2 by real-time PCR.

2-3-5
Bone marrow contents were collected from the femoral and tibial diaphyses of C57BL/6J mice (8 weeks old), and blood cell components and macrophage components were removed therefrom to obtain bone-marrow-derived mesenchymal stem cells. The cells were seeded in a 24-well plate at a concentration of 8.0 × 10⁴ cells/well, and the MHP1 peptide was added at a concentration of 4 mg/ml (25 µl per well), followed by culturing the cells for 24 hours. Osteogenic differentiation was induced by adding 2 ng/mL TGF-β, 10 mM β-glycerophosphate, and 50 µg/mL ascorbic acid, and ALP staining and real-time PCR were performed on day 4 for evaluation.

2-3-6
Bone marrow contents were collected from the femoral and tibial diaphyses of C57BL/6J mice (8 weeks old), and blood cell components and macrophage components were removed therefrom to obtain bone-marrow-derived mesenchymal stem cells. The cells were seeded in a 24-well plate at a concentration of 8.0 × 10⁴ cell/well, and the MHP1 peptide was added at a concentration of 4 mg/ml (25 µl per well), followed by culturing the cells for 24 hours. Osteogenic differentiation was induced by adding 2 ng/mL TGF-β, 10 mM β-glycerophosphate, and 50 µg/mL ascorbic acid, and evaluation was performed on day 20 by Alizarin red staining and quantification by absorbance measurement.

2-3-7
Human umbilical vein endothelial cells (HUVECs) were seeded in a 24-well plate at a concentration of 8.0 × 10⁴ cell/well, and cultured until confluent. A single line was drawn using the tip of a pipette tip, and the MHP1 peptide was added at a concentration of 4 mg/ml (25 µl per well). After culturing for 24 hours, the range of cell migration was quantitatively evaluated.

### 2-4: Results

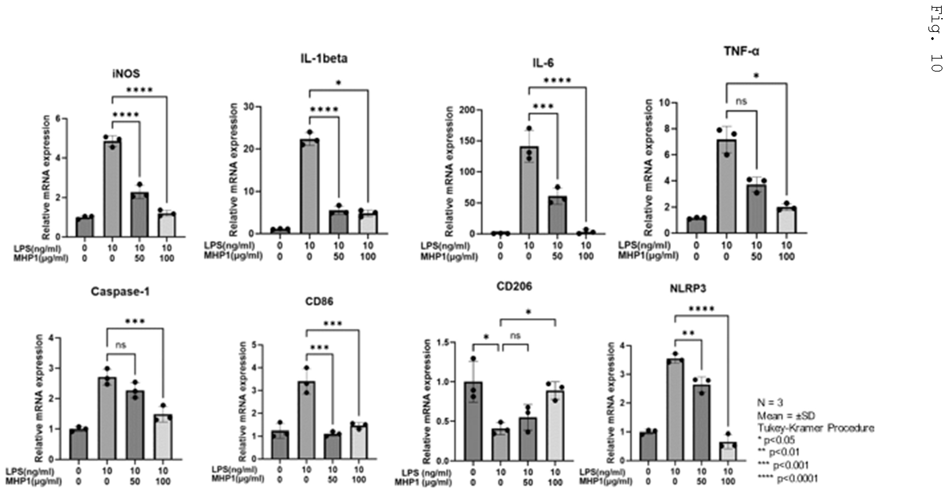

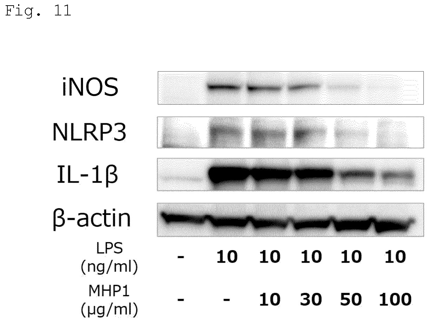

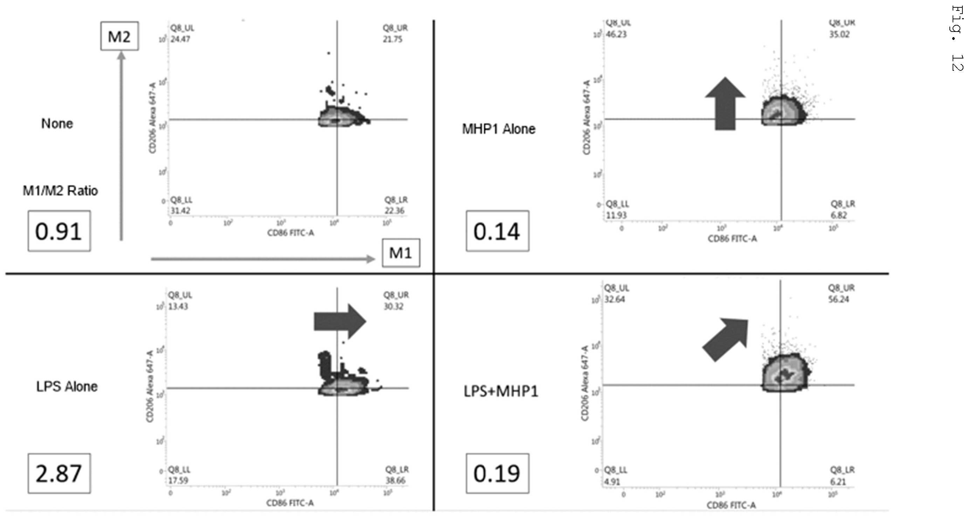

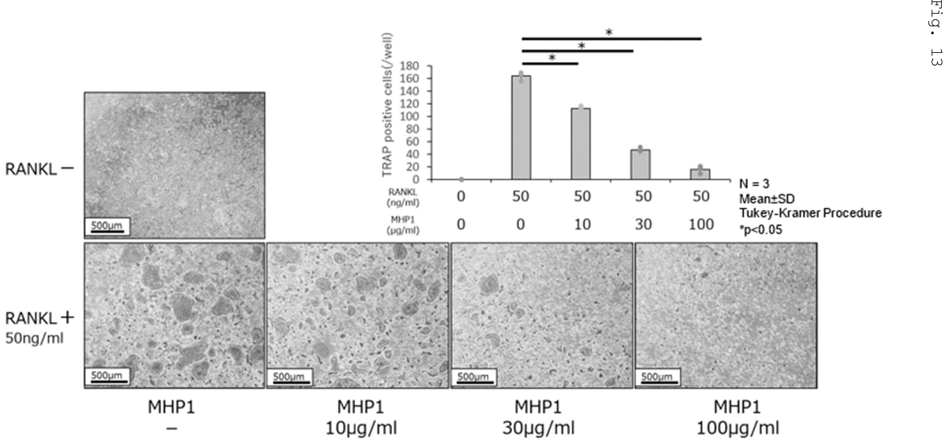

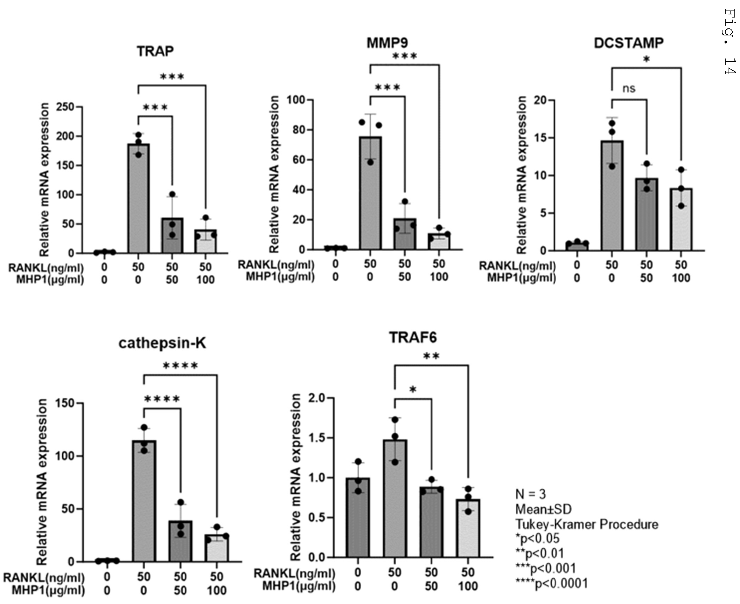

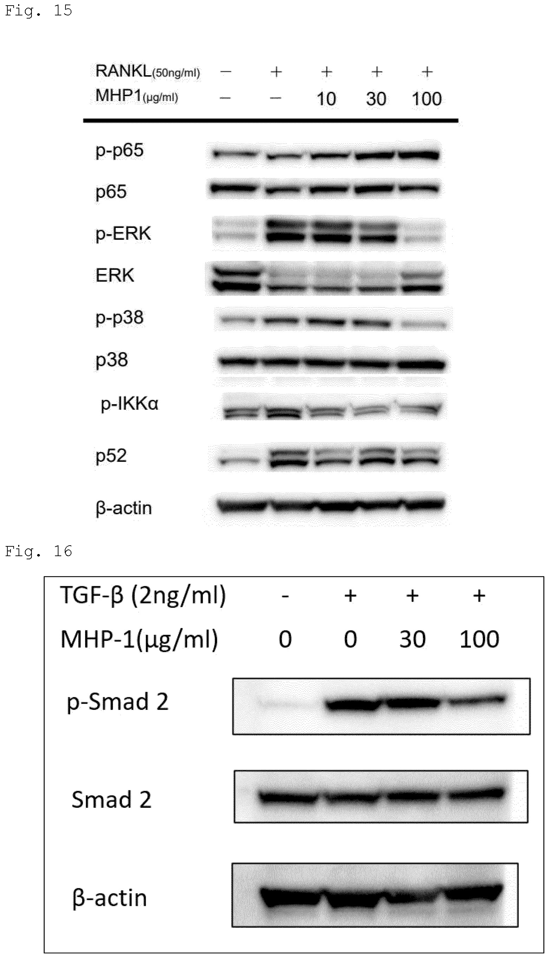

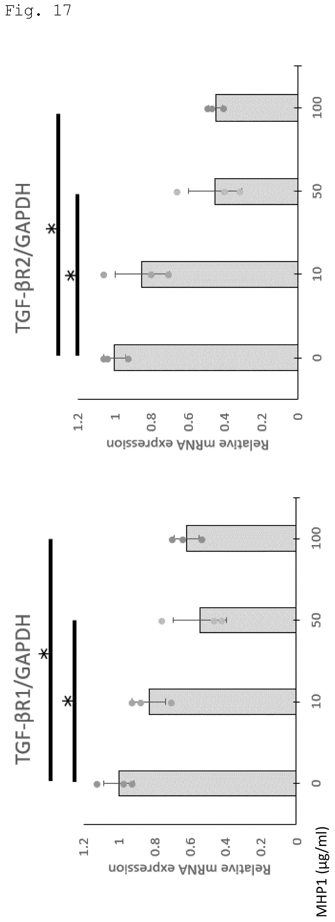

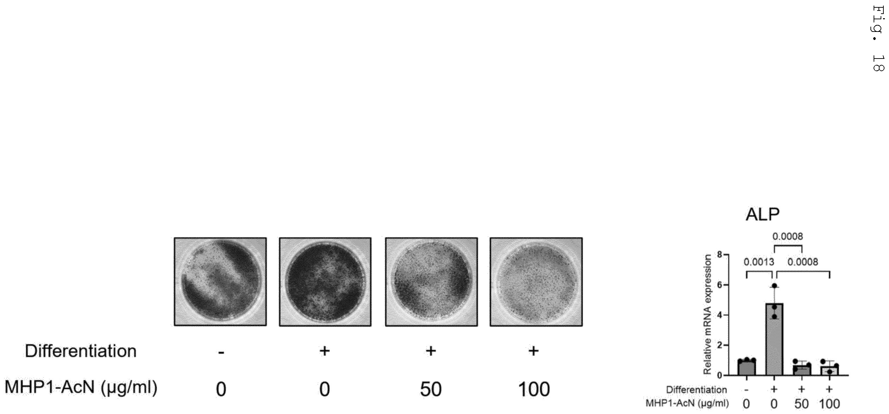

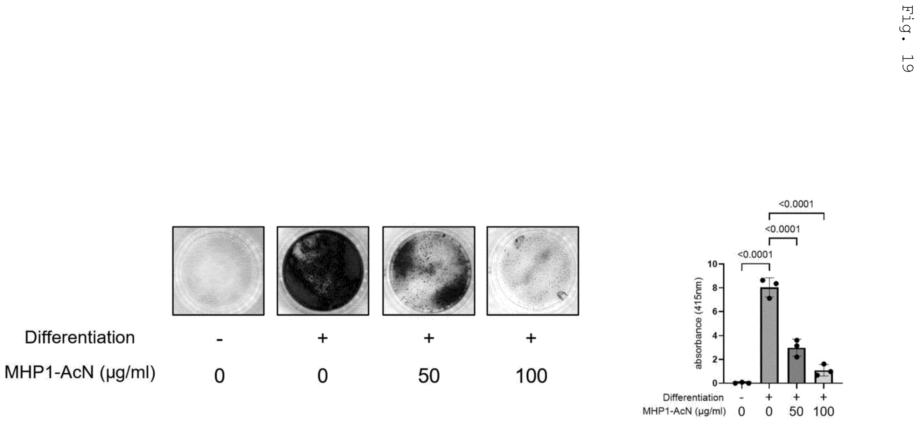

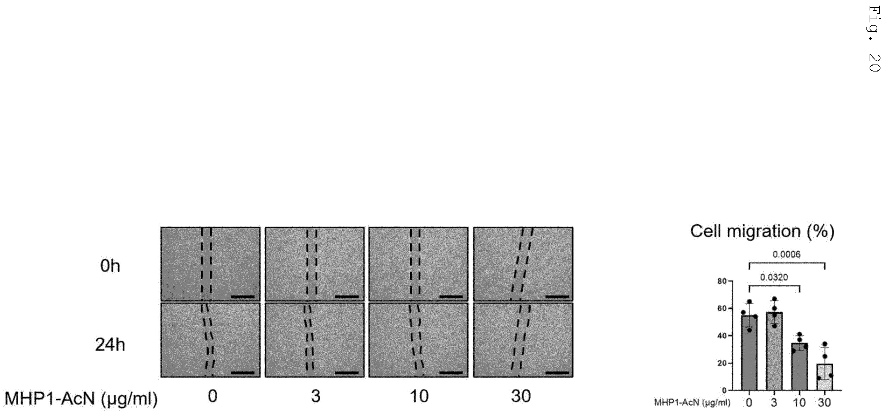

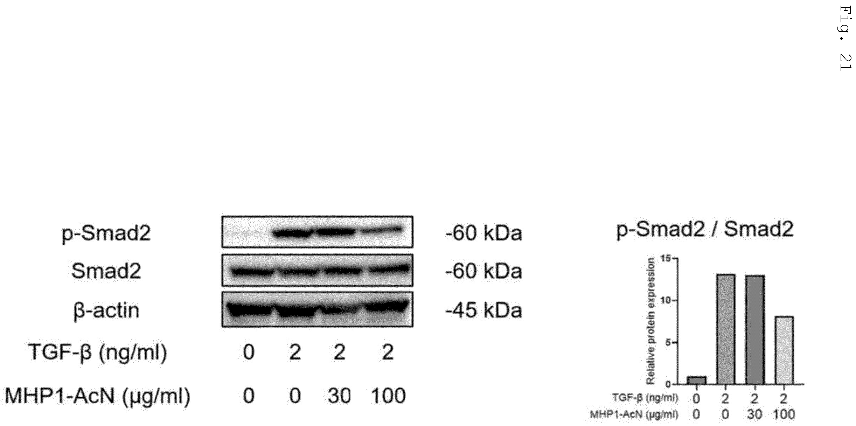

Figs. 10 to 21 show the results. Figs. 10 to 12 indicated that the RANKL peptide reduces the response of bone-marrow-derived macrophages to LPS stimulation and suppresses polarization to M1 and elevation of inflammatory cytokines. Figs. 13 to 15 indicated that the RANKL peptide suppresses differentiation of bone-marrow-derived macrophages into osteoclasts. Figs. 16 and 17 indicated that the RANKL peptide reduces TGF-β stimulation in bone-marrow-derived mesenchymal stem cells. Figs. 18 and 19 indicated that the RANKL peptide suppresses bone formation in bone-marrow-derived mesenchymal stem cells. Figs. 20 and 21 indicated that the RANKL peptide reduces cell migration of HUVECs.

## Claims

1. A prophylactic or therapeutic agent for osteoarthritis, comprising
an oligopeptide comprising
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
(a) the amino acid sequence of SEQ ID NO: 1, or
(b) an amino acid sequence in which one amino acid is substituted, deleted, added, or inserted with respect to the amino acid sequence of SEQ ID NO: 1, and
the β-strand D sequence is the following (c) or (d):
(c) the amino acid sequence of any of SEQ ID NOs: 2 to 5, or
(d) an amino acid sequence in which one to three amino acids are substituted, deleted, added, or inserted with respect to the amino acid sequence of any of SEQ ID NOs: 2 to 5.

2. The prophylactic or therapeutic agent according to claim 1, wherein the DE loop sequence is the amino acid sequence (a).

3. The prophylactic or therapeutic agent according to claim 2, wherein the one to three amino acids are one or two amino acids.

4. The prophylactic or therapeutic agent according to claim 1, wherein in the amino acid sequence (d), the N-terminal leucine residue of SEQ ID NOs: 2 and 4 and the leucine residue that is the 4th amino acid residue from the N-terminus of SEQ ID NOs: 3 and 5 are not substituted or deleted.

5. The prophylactic or therapeutic agent according to claim 1, wherein the amino acid sequence of the oligopeptide contains a β-strand E sequence of the RANKL protein, the β-strand E sequence being located adjacent to the C-terminal side of the DE loop sequence.

6. The prophylactic or therapeutic agent according to claim 5, wherein the β-strand E sequence is the following (e) or (f):
(e) the amino acid sequence of any of SEQ ID NOs: 6 to 9, or
(f) an amino acid sequence in which one to three amino acids are substituted, deleted, added, or inserted with respect to the amino acid sequence of any of SEQ ID NOs: 6 to 9.

7. The prophylactic or therapeutic agent according to claim 1, wherein the amino acid sequence of the oligopeptide does not comprise a CD loop sequence of the RANKL protein.

8. The prophylactic or therapeutic agent according to claim 1, wherein the amino acid sequence of the oligopeptide is the following (i) or (j):
(i) the amino acid sequence of any of SEQ ID NOs: 12 to 21, or
(j) an amino acid sequence in which one to three amino acids are substituted, deleted, added, or inserted with respect to the amino acid sequence of any of SEQ ID NOs: 12 to 21.

9. The prophylactic or therapeutic agent according to claim 1, wherein the amino acid sequence of the oligopeptide is the following (i') or (j'):
(i') the amino acid sequence of SEQ ID NO: 12, 17, 20, or 21, or
(j') an amino acid sequence in which one to three amino acids are substituted, deleted, added, or inserted with respect to the amino acid sequence of SEQ ID NO: 12, 17, 20, or 21.

10. The prophylactic or therapeutic agent according to any one of claims 1 to 9, wherein the oligopeptide has a length of 50 amino acid residues or less.

11. The prophylactic or therapeutic agent according to claim 10, wherein the oligopeptide has a length of 40 amino acid residues or less.

12. The prophylactic or therapeutic agent according to any one of claims 1 to 9, wherein the osteoarthritis is osteoarthritis in at least one joint selected from the group consisting of knee joint, shoulder joint, elbow joint, hip joint, ankle joint, and finger joint.

13. An osteosclerosis inhibitor, comprising
an oligopeptide comprising
an amino acid sequence containing
a DE loop sequence of RANKL protein, and
a β-strand D sequence of the RANKL protein, the β-strand D sequence being located adjacent to the N-terminal side of the DE loop sequence,
wherein
the DE loop sequence is the following (a) or (b):
(a) the amino acid sequence of SEQ ID NO: 1, or
(b) an amino acid sequence in which one amino acid is substituted, deleted, added, or inserted with respect to the amino acid sequence of SEQ ID NO: 1, and
the β-strand D sequence is the following (c) or (d):
(c) the amino acid sequence of any of SEQ ID NOs: 2 to 5, or
(d) an amino acid sequence in which one to three amino acids are substituted, deleted, added, or inserted with respect to the amino acid sequence of any of SEQ ID NOs: 2 to 5.
